Europäisches Patentamt

European Patent Office

Office européen des brevets

(11) Veröffentlichungsnummer: **0 343 520**
**A2**

## (12) EUROPÄISCHE PATENTANMELDUNG

(21) Anmeldenummer: **89108993.0**

(51) Int. Cl.⁴: **A61M 25/00**

(22) Anmeldetag: **19.05.89**

(30) Priorität: 25.05.88 DE 8806818 U

(43) Veröffentlichungstag der Anmeldung:
**29.11.89 Patentblatt 89/48**

(84) Benannte Vertragsstaaten:
**AT BE CH DE ES FR GB IT LI NL SE**

(71) Anmelder: **Schnepp-Pesch, Wolfram**
**Schönblick 6**
**D-7505 Ettlingen(DE)**

Anmelder: **Lindenberg, Josef**
**Käthe-Kollwitz-Strasse 10a**
**D-7500 Karlsruhe 1(DE)**

(72) Erfinder: **Schnepp-Pesch, Wolfram**
**Schönblick 6**
**D-7505 Ettlingen(DE)**
Erfinder: **Lindenberg, Josef**
**Käthe-Kollwitz-Strasse 10a**
**D-7500 Karlsruhe 1(DE)**

(74) Vertreter: **Dr.-Ing. Hans Lichti Dipl.-Ing. Heiner**
**Lichti Dipl.-Phys. Dr. Jost Lempert**
**Postfach 41 07 60 Durlacher Strasse 31**
**D-7500 Karlsruhe 41(DE)**

(54) **Ureter-Katheter-set.**

(57) Es wird ein Ureter-Katheter-Set mit mindestens einem Ureter-Katheter vorgeschlagen, das einen Verlängerungsadapter (6,16) mit einem in den Katheter (1) reibschlüssig einsetzbaren Adapterteil (7,17) mit einem dem Innendurchmesser des Katheters (1) entsprechenden Außendurchmesser, und einen Verlängerungskatheter (9) aufweist.

EP 0 343 520 A2

**Ureter-Katheter-Set**

Die Erfindung betrifft ein Ureter-Katheter-Set mit mindestens einem Ureter-Katheter. Es sind Ureter-Katheter bekannt. Diese weisen regelmäßig eine übliche Länge von etwa 70 cm auf. Wenn der Arzt nach Legen eines solchen Ureter-Katheters zur Durchführung einer Röntgenaufnahme der Niere durch diesen Katheter das Kontrastmittel einspritzt, so besteht die Gefahr, daß seine das Mittel einspritzende Hand im Bestrahlungsbereich des Röntgengeräts liegt, da der genannte kurze Katheter nur geringfügig aus der Urethra herausragt. Die Vorsichtsmaßnahme der Verlängerung eines längeren Katheters wird in der Regel nicht verwendet, da hierzu der eigentliche Ureter-Katheter entfernt und ein neuer Katheter gelegt werden müßte, was wiederum sehr zeitaufwendig und schwierig ist und darüberhinaus eine Belastung für den Patienten beinhaltet sowie die Gefahr von Perforationen erhöht. Ein Herausnehmen eines Ureter-Katheters und Legen neuer Führungsmittel muß aber durchgeführt werden, wenn Manipulationen erfolgen sollen, wenn beispielsweise eine Schleuse, ein Urethrorenoskop, ein Dilatationsbesteck, wie Stufenbougierungsbesteck etc. über ein Führungsmittel hin eingebracht werden soll. Auch hierbei sind die vorgenannten Nachteile im Hinblick auf Zeitaufwand, Belastung des Patienten und Gefahr von Perforationen gegeben.

Der Erfindung liegt daher die Aufgabe zugrunde, ein Ureter-Katheter-Set zu schaffen, bei dem die vorstehenden Nachteile vermieden und ein einmal gelegter Ureter-Katheter der üblichen Art liegen gelassen und bei weiteren Maßnahmen, wie Einbringen von Schleusen, Urethrorenoskopen, Dilatationsbestecken etc., aber auch bei Röntgenaufnahmen und zum Einspritzen des Kontrastmittel verwendet werden können, wobei im letzteren Fall der Strahlenschutz erhöht werden soll.

Erfindungsgemäß wird die genannte Aufgabe durch ein Ureter-Katheter-Set mit mindestens einem Ureter-Katheter gelöst, welches darüberhinaus ausgestaltet ist durch einen Verlängerungsadapter mit einem in den Katheter reibschlüssig einsetzbaren Adapterteil, mit einem dem Innendurchmesser des Katheters entsprechenden Außendurchmesser und mit einem Verlängerungskatheter. Durch die erfindungsgemäße Ausgestaltung eines Ureter-Katheter-Sets kann der Arzt an dem liegenden Ureter-Katheter den Verlängerungsadapter fest ansetzen und diesen dann mit dem zusätzlichen Verlängerungskatheter versehen, der in bevorzugter Ausgestaltung mit dem eigentlichen Ureter-Katheter identisch ist. Es kann also als Verlängerungskatheter ein entsprechender Ureter-Katheter verwendet werden.

Eine weitere bevorzugte Ausgestaltung sieht vor, daß auf dem Adapterteil zentral ein Zwischenteil fest aufsitzt, das stirnseitig von den Enden des Adapterteils überragt wird und das einen den Kathetern entsprechenden Außendurchmesser aufweist. Zur Verbesserung der Reibhaftung kann weiterhin vorgesehen sein, daß das Adapterteil an seinen Enden Profilierungen, gegebenenfalls in Gewindeform aufweist. Während bei den Einsatzzwecken des erfindungsgemäßen Ureter-Katheter-Sets nach Zusammensetzen als Führungsset für Schleusen etc. der Verlängerungsadapter massiv ausgebildet sein kann, sieht eine bevorzugte Ausgestaltung vor, daß das Adapterteil als Röhrchen mit durchgehendem Hohlraum ausgebildet ist, um das Einspritzen des Kontrastmittels bei einer Röntgenuntersuchung zu ermöglichen. Um auch ein derart ausgebildetes Ureter-Katheter-Set mit hohlem Adapterteil zum Einsatz als Führungsmittel für Schleusen etc. bequem aussteifen zu können, sieht eine weitere Ausbildung vor, daß der Hohlraum des Adapterteils sich zu den stirnseitigen Enden desselben über einen Teil seiner Länge sich konisch erweitert. Durch diese Ausgestaltung kann ein Versteifungsstilett, das eine etwa der Länge zweier der Katheter entsprechende Länge aufweist, bequem und ohne Schwierigkeiten durch den Verlängerungsadapter hindurchgesteckt werden, da es durch die konische Endausbildung des Hohlraums desselben bzw. seines Verlängerungsteils in den Hohlraum eingeführt wird.

Bei der Verwendung als Führungsteil kann das erfindungsgemäße Ureter-Katheter-Set weiterhin ausgebildet sein durch das das Adapterteil beidseitig überragende Verlängerungsstilett, wobei insbesondere beide Teile eines Verlängerungsstiletts einteilig miteinander ausgebildet sind. Insgesamt bietet das erfindungsgemäße Ureter-Katheter-Stilett eine Erleichterung und Vereinfachung beim Gebrauch von Ureter-Kathetern unter Reduzierung der oben erwähnten Gefahren.

Weitere Vorteile und Merkmale der Erfindung ergeben sich aus den Ansprüchen und aus der nachfolgenden Beschreibung, in der Ausführungsbeispiele unter Bezugnahme auf die Zeichnungen im einzelnen erläutert sind. Dabei zeigt:

Figur 1 Eine erste Ausgestaltung eines erfindungsgemäßen Ureter-Katheter-Sets;

Figur 2 eine gegenüber der Abbildung der Figur 1 abgewandelte Ausgestaltung eines Verlängerungsadapters als Teil eines erfindungsgemäßen Ureter-Katheter-Sets; und

Figur 3 eine weitere Ausgestaltung eines erfindungsgemäßen Ureter-Katheter-Sets.

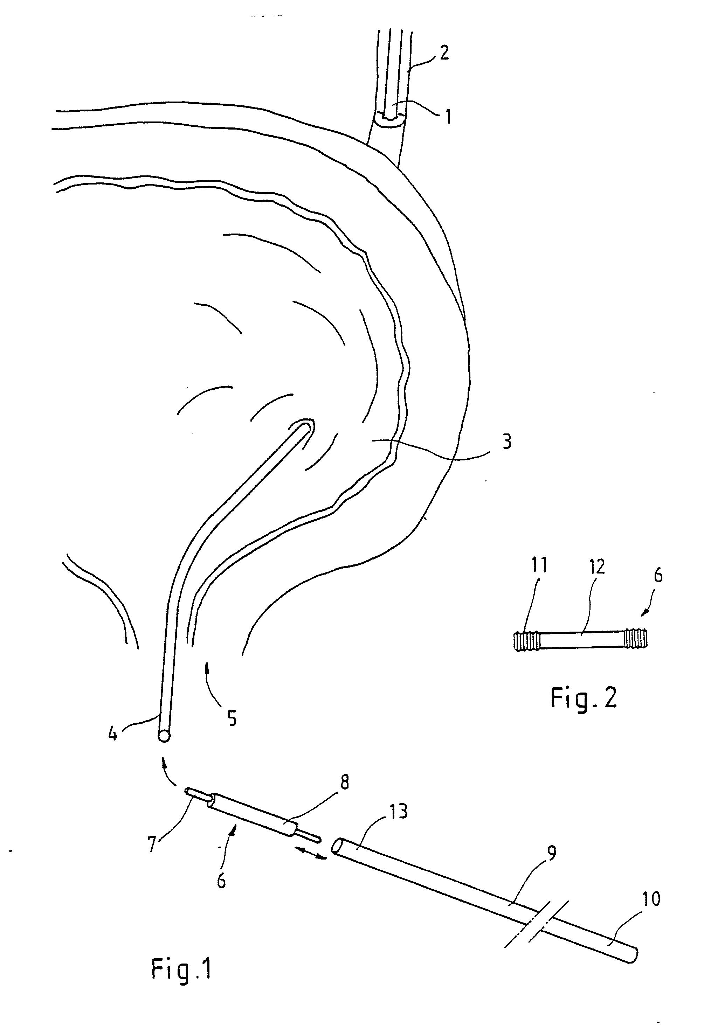

Das erfindungsgemäße Ureter-Katheter-Set weist zunächst einen Ureter-Katheter 1 auf, der in der Figur 1 in Situs dargestellt ist, das heißt, er liegt im Ureter 2, erstreckt sich durch die Vesica Urinalis 3 und ragt mit seinem proximalen Ende 4 aus der Urethra (bei 5) heraus. Weiterhin weist das erfindungsgemäße Ureter-Katheter-Set einen Verlängerungsadapter 6 auf, der im dargestellten Ausführungsbeispiel aus einem (hohlen) Metallröhrchen 7 besteht, auf dem ein Zwischenteil 8 aus Kunststoffmaterial aufsitzt. Das Metallröhrchen 7 überragt das Zwischenteil 8 an beiden Stirnseiten. Der Außendurchmesser des Metallröhrchen 7 entspricht dem Innendurchmesser eines Ureter-Katheters 1, so daß die überragenden Enden des Metallröhrchens 7 reibschlüssig in den Katheter 1 eingesetzt werden können. Das Kunststoffmaterial des Zwischenteils 8 ist vorzugsweise das gleiche Material wie das des Katheters 1. Weiterhin ist ein Verlängerungskatheter 9 vorgesehen, der ebenfalls durch Dimentionierung der entsprechenden Querschnittsabmessungen reibschlüssig auf ein Verlängerungsende des Verlängerungsadapters 6 aufsetzbar ist. Der Verlängerungskatheter 9 kann insbesondere ein gleicher Katheter sein wie der Ureter-Katheter 1.

Bei dem erfindungsgemäßen Ureter-Katheter-Set kann zunächst der Ureter-Katheter 1 in üblicher Weise gelegt werden. Durch die erfindungsgemäße Ausgestaltung braucht ein gelegter Ureter-Katheter1 beispielsweise zur Röntgenuntersuchung der Niere nicht mehr entfernt und ein weiterer Katheter gelegt werden, was schwierig, aufwendig und mit Gefahren für den Patienten sowie Belastungen verbunden ist; vielmehr wird der Verlängerungsadapter 6 lediglich mit dem proximalen Ende 4 des Katheters 1 verbunden und anschließend der Verlängerungskatheter 9 auf die andere Stirnseite des Verlängerungsadapters 6 aufgesteckt wobei vom proximalen Ende 10 des Verlängerungskatheters 9 Kontrastmittel durch diesen, das Röhrchen 7 des Adapters 6 und den eigentlichen Ureter-Katheter 1 in die Niere eingespritzt werden.

Aufgrund der Fließfähigkeit der Kontrastmittel ist eine Druckreduzierung nicht zu befürchten. Neben den vorgenannten Vorteilen ergibt sich als weiterer, wesentlicher Vorteil, daß die Hand des Arztes, mit der er das Kontrastmittel einspritzt, während der Röntgenaufnahme weiter fort von dem bestrahlten Bereich ist, so daß die Gefahr einer Bestrahlung der Hand des Arztes wesentlich vermindert bzw. sogar ausgeschaltet wird, während bisher hier größere Belastungen aufgrund einer Vielzahl von Röntgenuntersuchungen an einem Behandlungstag nicht auszuschließen waren.

Statt der bevorzugten Ausgestaltung des Verlängerungsadapters 6 der Figur 1 mit einem glatten Röhrchen 7 und einem auf diesem aufsitzenden Zwischenteil kann auch vorgesehen sein, daß die Enden des Röhrchens 7 mit Außengewinden 11 versehen sind, um die Haftung in den Kathetern 1,9 zu verbessern, und/oder der Adapter 6 lediglich aus einem Röhrchenteil 12 besteht, wie dies in der Figur 2 dargestellt ist, wobei die beiden Katheter 1,9 dann mit ihrem proximalen Ende 4 bzw. ihrem distalen Ende 13 so weit über das Röhrchen 12 geschoben werden, bis sie sich mit ihren Stirnseiten berühren.

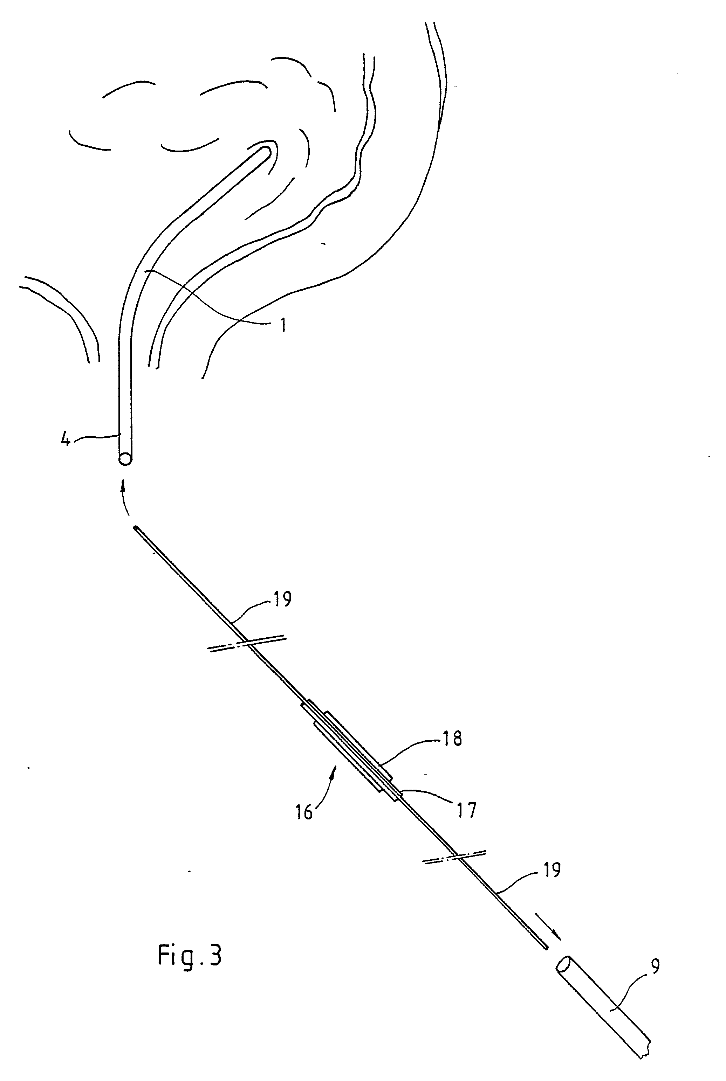

Eine weitere Ausgestaltung eines erfindungsgemäßen Ureter-Katheter-Sets ist in der Figur 3 dargestellt. Auch dieses Set weist wieder einen Ureter-Katheter 1 auf, der nach Legen mit seinem proximalen Ende 4 aus der Urethra hinausragt. Weiterhin weist dieses Set wieder einen Verlängerungskatheter 9 auf.

Der Verlängerungsadapter 16 weist wiederum ein Adapterteil 17 auf, das einen dem Innendurchmesser das Katheter 1,9 entsprechenden Außendurchmesser aufweist. Auf dem Adapterteil 17, das vorzugsweise aus Metall besteht, sitzt weiterhin ein Zwischenteil 18 mittig auf, das von den Enden des Adapterteils 17 überragt wird. Weiterhin wird das Adapterteil 17 beidseitig durch Verlängerungsstiletts 19 überragt. Es kann sich hierbei um ein einstückiges Verlängerungsstilett 19 handeln, das sich durch das Adapterteil 17 hindurch erstreckt und mit diesem fest ver bunden ist. Alternativ kann das Adapterteil 17 auch einstückig mit dem Verlängerungsstilett 19 als verstärkter Bereich desselben ausgebildet sein. Weiterhin könnten in ein separates Adapterteil 17 auch von beiden Seiten her einzelne Stiletts 19 eingesteckt und mit dem Adapterteil 17 verbunden sein. Das Stilett 19 überragt das Zwischenteil 18 beidseitig gerade um etwa die Länge eines Katheters 1,9. Weiterhin könnte ein einstückiges Stilett mit einer etwa der Länge zweier Katheter entsprechenden Länge vorgesehen sein, um durch einen Verlängerungsadapter nach den Figuren 1, 2 hindurchgesteckt zu werden, um so den Einsatzzwecken der Ausgestaltung der Figur 3 zu genügen.

Auch bei der Ausgestaltung der Figur 3 kann der einmal gelegte Ureter-Katheter 1 liegen bleiben und muß nicht entfernt werden, um beispielsweise eine Schleuse einzubringen, ein Urethrorenoskop, Dilatationsbestecke, beispielsweise in Stufenbougierungsbestecke einzubringen. Stattdessen ermöglicht diese Ausgestaltung, daß das distale Stilettteil 19 in das proximale Ende 4 des Ureter-Katheters 1 eingeführt und schließlich das Adapterteil 17 reibschlüssig in das distale Ende 4 des Katheters 1 eingesetzt wird. Anschließend kann auf das andere Stilettteil der Verlängerungskatheter 9 aufgeschoben und reibschlüssig auf dem Adapterteil 17 aufgesteckt werden. Der durch das Stilett 19 versteifte Doppelkatheter 1,9 kann dann als Führungsmittel für die erwähnten Schleusen, ein Urethrorenoskop, Dilatationsbestecke etc. dienen, die über dieses in die Harnröhre und bis zur Niere geschoben werden. Durch die reibschlüssige Verbindung der Katheter mit dem Adapterteil kann anschließend das gesamte miteinander verbundene, erfindungsgemäße Ureter-Katheter-Set aus dem eingeschobenen Teil, wie einer Schleuse, einem Dilatationsbesteckt od.dgl. herausgezogen werden.

**Ansprüche**

1. Ureter-Katheter-Set mit mindestens einem Ureter-Katheter, gekennzeichnet durch einen Verlängerungsadapter (6,16) mit einem in den Katheter (1) reibschlüssig einsetzbaren Adapterteil (7,17) mit einem dem Innendurchmesser des Katheters (1) entsprechenden Außendurchmesser, und durch einen Verlängerungskatheter (9).

2. Katheter-Set nach Anspruch 1, dadurch gekennzeichnet, daß Ureter-Katheter (1) und Verlängerungskatheter (9) identisch ausgebildet sind.

3. Katheter-Set nach Anspruch 1 oder 2, dadurch gekennzeichnet, daß auf dem Adapterteil (7,17) zentral ein Zwischenteil (8,18) fest aufsitzt, das stirnseitig von den Enden des Adapterteils (7,17) überragt wird und das einen den Kathetern (1,9) entsprechenden Außendurchmesser aufweist.

4. Katheter-Set nach einem der Ansprüche 1 bis 3, dadurch gekennzeichnet, daß das Adapterteil (7,17) an seinen Enden Profilierungen, gegebenenfalls in Gewindeform aufweist.

5. Katheter-Set nach einem der Ansprüche 1 bis 4, dadurch gekennzeichnet, daß das Adapterteil (7) als Röhrchen mit durchgehendem Hohlraum ausgebildet ist.

6. Katheter-Set nach Anspruch 5, dadurch gekennzeichnet, daß der Hohlraum des Adapterteils (7) sich zu den stirnseitigen Enden desselben über einen Teil seiner Länge sich konisch erweitert.

7. Katheter-Set nach einem der vorangehenden Ansprüche, gekennzeichnet durch das das Adapterteil (17) beidseitig überragende Verlängerungsstilett (19).

8. Katheter-Set nach Anspruch 7, dadurch gekennzeichnet, daß beide Teile eines Verlängerungsstiletts (19) einteilig miteinander ausgebildet sind.

Fig.1

Fig. 2

Fig.3